Europäisches Patentamt

European Patent Office

Office européen des brevets

(11) Publication number: **0 576 039 A2**

(12) **EUROPEAN PATENT APPLICATION**

(21) Application number: 93113126.2

(22) Date of filing: **05.07.88**

(51) Int. Cl.5: **C12Q 1/56**

This application was filed on 17 - 08 - 1993 as a divisional application to the application mentioned under INID code 60.

(30) Priority: **06.07.87 US 70068**

(43) Date of publication of application:
**29.12.93 Bulletin 93/52**

(60) Publication number of the earlier application in accordance with Art.76 EPC: **0 324 027**

(84) Designated Contracting States:
**AT BE CH DE FR GB IT LI LU NL SE**

(71) Applicant: **Biopool International, Inc.**
**6025 Nicolle Street**
**Suite A**
**Ventura, CA 93003(US)**

(72) Inventor: **Ranby, Mats Gustaf**
**Biologigrand 10**
**S-904 41 Umea(SE)**
Inventor: **Wiman, Tor-Bjorn**
**Porsvagen 364**
**S-191 48 Sollentuna(SE)**

(74) Representative: **Sternagel, Hans-Günther, Dr.**
**et al**
**Patentanwälte Dr. Michael Hann, Dr. H.-G.**
**Sternagel, Dr. H. Dörries,**
**Sander Aue 30**
**D-51465 Bergisch Gladbach (DE)**

(54) Method of determining tissue plasminogen activator activity in biological fluids.

(57) The present invention relates to an improved method for measuring tissue plasminogen activator and plasminogen activator inhibitor. The method utilizes a genetically modified tissue plasminogen activator protein as a substrate. The present invention also relates to improved methods of collecting blood so that endogenous plasminogen activator is not inactivated.

EP 0 576 039 A2

Rank Xerox (UK) Business Services
(3.10/3.6/3.3.1)

**Technical Field**

The present invention relates to an improved method for measuring tissue plasminogen activator, and plasminogen activator inhibitor, and soluble fibrin. More particularly, the present invention relates to a method for measuring plasminogen activator inhibitor and soluble fibrin which utilizes a genetically modified tissue plasminogen activator protein as a reagent. The present invention also relates to improved methods of collecting blood so that endogenous plasminogen activator is not inactivated.

**Background**

The term "t-PA" means "tissue plasminogen activator". The term "PAI" means plasminogen activator inhibitor. "PAI 1" is plasminogen activator inhibitor one and is sometimes called endothelial plasminogen activator inhibitor. "PAI 2", is plasminogen activator inhibitor two and is sometimes called placental plasminogen activator inhibitor. The term "$\alpha_2$AP" means alpha 2 antiplasmin and is a protein found in blood of normal individuals that inhibits the enzyme plasmin. The term "fibrinogen digests" includes the products from digestion of fibrinogen or fibrin with proteolytic enzymes or plasmin.

Investigation of tissue plasminogen activator inactivation in plasma has been hampered by poor methodology. A specific and sensitive method for measuring t-PA in plasma samples where potential fibrinolytic inhibitors were neutralized by controlled acidification was described. (See Wiman, B., *et al., Clin. Chim Acta*, **127**,279-288,1982) t-PA subsequently measured by this method exhibited a parabolic rate assay. (See Rånby, M., *et al, Thromb, Res*. **27**, 743-749, 1982). With this method it was also possible to specifically determine inhibitory activity to t-PA in plasma and kinetic evidence for a fast t-PA inhibitor in plasma was presented.

Assuming the formation of a stoichiometric 1:1 complex, a rate constant of about $10^7$ $M^{-1} \cdot s^{-1}$ was calculated, and the plasma concentration of the new inhibitor in healthy individuals was determined as 8 ± 2 unit/ml (1 unit = inhibition of 1 international unit of t-PA) The t-PA inhibitory content was also determined in plasma from various patients. High inhibitory activity content was frequently found in patients with deep venous thombosis, hemostatic problems during late pregnancy, or severe coronary heart disease. (See Chmielewska, J., *et al., Thromb. Res*. **31**, 427-436 (1983). The PAI activity observed is now known to be the result of PAI 1 activity.

There are several assays systems that are commercially available for the measurement of t-PA and PAI which utilize native t-PA that has been isolated from melanoma cells. However, the results concerning PAI levels obtained from these assays are not always reliable because of the cleavage of t-PA into the two chain form of the protein. Native one-chain t-PA is cleaved by plasmin or by trypsin after the Arg in the sequence -Gln-Phe-Arg-Ile-Lys- in the t-PA protein. This creates a problem when trying to measure the PAI 1 level in a biological fluid by inhibition of the t-PA activity because two chain t-PA also reacts rapidly with PAI 2 and reacts much faster than single chain t-PA with other protease inhibitors such as $\alpha_2$AP

Thus, what is needed is a t-PA that is resistant to cleavage by proteolytic enzymes to prevent formation of two chain t-PA in the preparation procedure or during the assay procedure. With such a t-PA, the presence of PAI could be more accurately measured.

Another problem encountered in the prior art methods of measuring t-PA and PAI is the fact that t-PA and PAI 1 activity are quite unstable after blood is collected and the activities of the two proteins rapidly decreases after blood is collected.

When t-PA is assayed in blood, it has been found that polylysine is an effective activator of the t-PA. However, it has also been found that polylysine is not an effective activator of t-PA in biological fluids other than blood and blood plasma. Thus, what is needed is to identify the preparation in plasma that, together with polylysine, constitutes the effective activator of t-PA which will allow one to determine the t-PA level in non-plasma fluids.

Another problem encountered in the prior art is that plasma samples must be acidified and incubated for relatively long periods of time at low pH to destroy the plasmin inhibitory activity in the plasma sample that interferes with the assay. Due to inhibitors, tPA activity is not stable in blood or in blood plasma and hence cannot be determined accurately be determined in regular citrated plasma samples. What is needed is a method of stabilizing the activity of tPA so that a sample of blood can be collected and analyzed under normal procedures. When prior art methods of collecting blood are used, much of the tPA activity is lost before the blood can be analyzed.

Yet another problem encountered in the prior art of determining t-PA activity is that the t-PA activity is underestimated in biological fluids that also contain PAI 1 activity. This is because PAI 1 activity will exert its biological activity on the assay system. What is needed is a method of inhibiting PAI 1 activity in the assay system when measuring t-PA activity in biological fluids.

## Summary of the Invention

In accordance with the present invention, variants of one-chain t-PA where the -Arg- amino acid was replaced by a His (Arg to His) or by a Lys (Arg to Lys) or by a Thr (Arg to Thr) were made through genetic modification of the native t-PA protein. All the mutants, including the uncleavable Arg to Thr mutant could be used in determination of PAI activity in plasma samples. The Arg to Thr mutant represents an advantage in PAI 1 activity determination. Preparations of the Arg to Thr mutant are sure to be free of two chain t-PA. The two chain t-PA, in contrast to single chain t-PA, reacts readily with a second form of PAI known as PAI 2. Thus, by using the mutant t-PA one can accurately measure PAI 1, an inhibitor that selectively reacts with single chain t-PA.

In addition, the present invention encompasses an improved method of collecting blood so that the t-PA present in the blood is stabilized and is not readily inactivated by PAI present in the blood. The improved method of collecting blood comprises acidifying the blood from the physiological pH of about 7.3 to a pH of between approximately 4.0 to 6.0. The preferred method of acidifying the blood for assaying t-PA is with citrate buffer although it is to be understood that other buffers can be used in practicing the present invention. To reduce hemolysis of blood during the acidification, it has been found advantageous to add Pluronic® F-68 to the blood citrate buffer mixture.

Another aspect of the present invention is the discovery that fibrinogen is the plasma component that must be present for activation of t-PA by polylysine. Plasmin digests of fibrinogen or fibrin will also activate t-PA in the presence of polylysine. Thus, when t-PA is being measured in biological fluids other than blood, fibrinogen (or fibrinogen digestion products) must be added along with polylysine to obtain maximum stimulation of t-PA activity.

Accordingly, it is an object of the present invention to provide an improved method of assaying plasminogen activator inhibitor activity.

It is an object of the present invention to provide a t-PA that is resistant to cleavage by proteolytic enzymes and is useful in the assay of PAI 1.

It is another object of the present invention to provide a method of collecting blood or other biological fluid that will stabilize t-PA activity.

Another object of the present invention is to provide a method of immediately lowering the pH of the blood with a minimum of hemolysis.

It is yet another object of the present invention to provide a method of using polylysine in combination with fibrinogen or fibrinogen digests as a t-PA cofactor in non-blood biological fluids.

Yet another object of the present invention is to provide an improved method of measuring fibrin in a sample utilizing a single chain t-PA that is resistant to cleavage by proteolytic enzymes.

Another object of the present invention is to improve the assay of t-PA activity by including antibodies that inhibit PAI 1 and $\alpha_2$ antiplasmin activities since these activities interfere with the assay of t-PA activity.

These and other objects, features and advantages of the present invention will become apparent after a review of the following detailed description of the disclosed embodiment and the appended claims.

## Description of the Figure

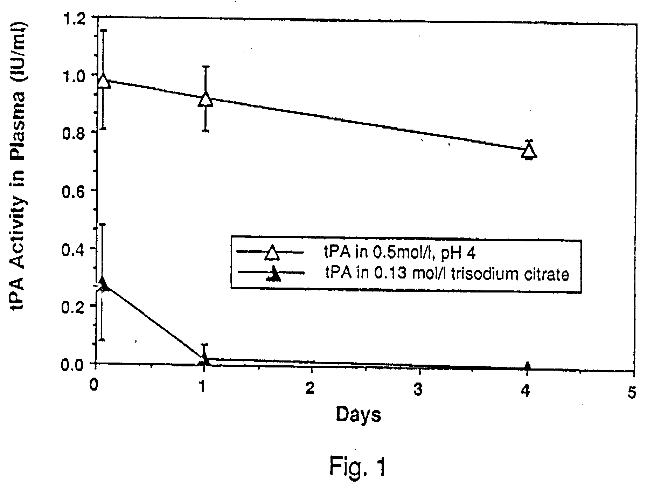

Fig. 1 shows tPA activity in blood collected in different buffers.

## Detailed Description of the Disclosed Embodiment

Native one-chain t-PA is cleaved by plasmin or by trypsin after the Arg in the sequence -Gln-Phe-Arg-Ile-Lys- in the t-PA protein. This creates a problem when trying to measure the PAI level in a biological fluid by inhibition of the t-PA activity. For example, t-PA activity is known to be stimulated by by fibrin or fibrin fragments. The stimulation of single chain t-PA by the presence of fibrin is much greater than that of the two chain t-PA.

The mutant t-PA reacted poorly with polyclonal antibodies raised against the peptide -Gln-Pro-Gln-Phe-Arg-Ile-Lys-Gly-Gly- but reacted readily with the native protein. The specific activity expressed in IU/$\mu$g were as follows: 810 (Arg to His), 640 (Arg to Lys), 290 (Arg to Thr) as compared to 810 for the wild type and 660 for Bowes melanoma t-PA. The amidolytic activity against D-Ile-Pro-Arg-pNA at 37°C, pH 9.0 expressed in mOD per minute at 1$\mu$g/ml of enzyme was 15.8 (Arg to His), 13.6 (Arg to Lys), 8.3 (Arg to Thr), 10.0 (wild type), 9.6 for melanoma one chain t-PA as compared to 55.2 for two chain melanoma t-PA.

All mutant t-PA's including the uncleavable Arg to Thr mutant could be used in determination of PAI activity in plasma samples. The Arg to Thr mutant represents a theoretical advantage in PAI 1 activity determination. Preparations of the Arg to Thr mutant are sure to be free of two chain t-PA which, in contrast to the single chain t-PA, also reacts readily with PAI 2.

It is to be understood that the genetically modified t-PA that is described herein are readily made by those of ordinary skill in the recombinant DNA art.

The plasminogen activation rate in the presence and absence of fibrin at 0.5 $\mu$m plasminogen and 37°C was measured and the stimulation factor calculated. This was about 950 fold for the Arg to Thr mutant which was considerably higher than that of melanoma one chain t-PA and the other mutant t-PA which were all about 550 fold. The stimulation factor for melanoma two chain t-PA was about 120 fold. It is to be understood that the extra fibrin sensitivity of the Arg to Thr mutant resulted in an improved soluble fibrin assay according to the Wiman-Rånby protocol (See Wiman, B and Rånby, M, *Thromb. Haemostas.* 55, 189-183(1986)). Thus, the plasmin insensitive protein-engineered mutant t-PA is shown to be advantageous over prior art methods when used in assays for PAI 1 activity and soluble fibrin.

It is to be understood that the t-PA activity measured according to the present invention can optionally be determined using a reagent containing antibodies that inhibit anti-plasmin activity and PAI activity present in the biological fluid to be analyzed.

The present invention also includes a method for determining the content of soluble fibrin in a sample comprising the steps of mixing a fixed amount of sample with a fixed amount of reagent containing one-chain t-PA, plasminogen and a plasmin substrate. measuring plasmin substrate cleavage correlating this rate with the amount of soluble fibrin in the sample.

It was found that if during blood sample collecting, the pH of the blood is immediately lowered from the physiological pH 7.3 to a pH of between approximately 4.0 and 6.0, several advantages for the stability of the fibrinolytic components could be gained without introducing any of the disadvantages or inconveniences found when compared to commonly used Vacutainer®, Venaject® systems for collecting blood.

According to the present invention, nine parts of blood is drawn into a container that contains one part citrate buffer with a pH of about 4 and a molarity of about 1 mol/l of citrate. The final pH of the blood sample should preferably be between approximately 4.5 to 6.5. The citrate buffer should preferably be between 0.5 to 2 mol/l of a sodium citrate buffer at a pH of approximately 4.0 to 5.5 to which 5 to 15 parts by volume of blood is added during collection.

To prevent hemolysis of the red blood cells and thrombocyte activation in the blood sample, Pluronic® F-68 (BASF Corporation. Parsippany, NJ) can optionally be included in the solution into which the blood is being collected. The optimal final concentration of Pluronic® F-68 is between approximately 0.01% to 0.1% (0.1 to 1 mg/ml). It is to be understood that other Pluronic® surfactants can be used in the present invention to prevent hemolysis.

The Pluronic® F-68 is a species within the following general formula:

$$HO(C_2H_4O)_b(C_3H_6O)_a(C_2H_4O)_bH$$

wherein a is an integer such that the hydrophobe represented by $(C_3H_6O)$ has a molecular weight of approximately 950 to 4000, preferably about 1750 to 3500, and b is an integer such that the hydrophile portion represented by $(C_2H_4O)$ constitutes approximately 50% to 90% by weight of the compound.

Pluronic® F-68 has the following specific formula:

$$HO(C_2H_4O)_b(C_3H_6O)_a(C_2H_4O)_bH$$

wherein the molecular weight of the hydrophobe $(C_3H_6O)$ is approximately 1750 and the total molecular weight of the compound is approximately 8400.

The advantages of the procedure are that the rates of the reactions between t-PA and PAI 1 and between t-PA and other inhibitors of blood are reduced at a pH of about 5 as compared to those at the physiological pH of about 7.3. These reduced reaction rates will greatly increase the stability of t-PA activity in plasma. This is most important since it is relevant to determine the t-PA activity at the time of sampling and not the lower t-PA activity found in stored blood samples collected according to the prior art. Thus, the present invention will improve the value of measuring t-PA activity to diagnose the etiology of thrombotic disease, the risk of developing thrombotic disease or the t-PA activity obtained during t-PA therapy.

The preferred method of collecting blood for measuring tPA activity comprises collecting the blood in a container containing a citrate buffer. The buffer comprises approximately 0.5 mol/l sodium citrate at a pH of approximately 4.0. It is to be understood that the citrate buffer can have another metal cation such as

4

potassium. Only moderate pH reduction is required to conserve tPA activity in the blood. Good tPA stability is found at a final pH range of approximately 5.3 to 5.8 with a preferred pH of approximately 5.5. These conditions are mild indicating that this approach may prove valuable for ther analytes, *e.g.*, fibrinogen, plasminogen, ATIII, protein C, etc.

It has been determined that the stability of PAI activity is increased significantly in blood samples collected according to the present invention as compared to those collected in a conventional way. This is of importance for this type of assay since the instability of PAI 1 activity is limiting the spread of this clinically important assay.

In addition, the method according to the present invention preserves soluble fibrin levels in blood and plasma samples thereby improving the diagnostic importance of this important assay. High levels of soluble fibrin are found in the blood of patients with malignancies, risk pregnancies and in patients suffering from severe trauma. High levels of soluble fibrin is also a symptom of disseminated intravascular coagulation.

Thus, the present invention of lowering the pH of blood during collection greatly improves the stability in the blood sample and in the plasma sample derived thereof of several important fibrinolytic parameters namely t-PA activity, PAI 1 activity and soluble fibrin.

It has been determined that in the use of poly-D-lysine stimulation for t-PA medicated plasminogen activation, it was found the human plasma contained a factor which increased the stimulating effect of poly-D-lysine. In this work, this factor was identified as fibrinogen and it was further found that the plasmin digestion products of fibrinogen or fibrin also had the effect. This discovery is important because it makes possible the formulation of practical and inexpensive reagents for determining t-PA activity in blood plasma and PAI1 activity in blood plasma. This is most important in the clinical routine.

**Example I**

The following example describes measurement of t-PA according to the present invention:

Citric acid monohydrate, $M_r$ = 210, and Tri-sodium citrate dihydrate, $M_r$ = 294, was obtained fro Merck Darnstadt, W.G. Pluronic® F-68 was obtained from BASF Corporation, Parsipanny, New Jersey. Blood was obtained by vein puncture and collected on 0.13 mol/l trisodium citrate (1 part citrate buffer to 9 parts blood) in a siliconized Venoject® collection tube.

0.5 mol/l solutions of citric acid and of tri-sodium citrate were mixed to give 0.5 mol/l sodium citrate solutions with pH of 4.0, 4.5, 5.0 and 5.5. Each of these solutions was aliquoted and 25% F68 was added to give final concentrations of 0, 0.1 or 1% by weight.

1.0 mol/e and 2.0 mol/e citrate buffers pH 4.0, 4.5, 5.0 and 5.5 containing 0,0.1 or 1% F68 were made accordingly.

Citrated blood was dispensed in 300 $\mu$l aliquoted to which 33 $\mu$l of each of the 36 different buffers were added, mixed and incubated at room temperature (22°C) for 20 hours. The samples were centrifuged six minutes at 1500 x g, diluted six fold in 0.15 mol/l NaCl all and subjected to pH and absorptivity at 537 nm determination. The absorption value (optical density at 1 cm path length) was multiplied by the dilution factor of 6.

To a series of blood sample aliquots 1:9 volume of various citrate buffers were added. Plasma was obtained some 20 hours later and the pH and 537 nm absorptivity was determined. The results are shown in Table 1.

Table 1 pH and absorption at 537 nm for plasma obtained from blood samples to which 1:9 volume of citrate buffers with various concentrations, pH and F68 content were added.

## Table 1

| pH | 0.5 mol/l Citrate | | | 1.0 mol/l Citrate | | | 2.0 mol/l Citrate | | |
|---|---|---|---|---|---|---|---|---|---|
| | Conc. F68 (mg/ml) | | | Conc. F68(mg/ml) | | | Conc. F68(mg/ml) | | |
| | 0 | 1 | 10 | 0 | 1 | 10 | 0 | 1 | 10 |
| 4.0 pH | 5.62 | 5.50 | 5.58 | 4.88 | 4.90 | 4.94 | 4.62 | 4.58 | 4.60 |
| A | 0.54 | 0.51 | 0.75 | 4.61 | 3.32 | 2.24 | 10.92 | 10.5 | 11.6 |
| 4.5 pH | 6.07 | 6.04 | 6.09 | 5.48 | 5.44 | 5.41 | 5.08 | 5.10 | 5.10 |
| A | 0.62 | 0.56 | 0.55 | 0.61 | 0.49 | 0.46 | 5.24 | 1.09 | 1.40 |
| 5.0 pH | 6.50 | 6.48 | 6.52 | 5.92 | 5.83 | 5.90 | 5.58 | 5.51 | 5.53 |
| A | 0.67 | 0.73 | 0.678 | 0.53 | 0.48 | 0.44 | 0.92 | 0.92 | 0.91 |
| 5.5 pH | 6.78 | 6.83 | 6.92 | 6.34 | 6.33 | 6.35 | 6.02 | 5.93 | 5.99 |
| A | 0.72 | 0.68 | 0.65 | 0.84 | 0.70 | --* | 0.51 | 1.10 | 1.29 |

* not determined

As can be seen from Table 1, the addition of F68 to the freshly collected blood reduced the hemolysis of the red blood cells as indicated by the absorbance at 537 nm. This particularly clear with the addition of 1 mol/l citrate at a pH of 4.0 and with the addition of 2 mol/l citrate at a pH of 4.5. In both cases, there is a dose dependent increase in the stability of the red blood cell membranes as shown by absorbance at 537 run decreases as the Pluronic® F-68 concentration increases. It should be noted that this experiment was performed with blood of an apparently healthy individual and problems with hemolysis were small. These problems can be expected to be much greater when large numbers of patient plasmas are sampled.

**Example 2**

Venoject®, Teruno Europe, Lewen, Belgium, are evacuated siliconized 4.5 ml tubes containing 0.45 ml 0.13 mol/l sodium citrate and are, in the following, called "Venoject regular". Some "Venoject regular" were modified as an embodiment of the present invention. The citrate buffer in the "Venoject regular" tubes is removed by suction with a hypodermic needle and 0.45 ml 1.0 mol/l citrate buffer pH 4.0 is introduced through the rubber stopper with a hypodermic needle. In this way, the citrate buffer content is changed without disturbing the vacuum in the tube. The modified tubes are hereinafter called "Venoject modified".

Blood is drawn by vein puncture from an apparently healthy individual into two "Venoject regular" and into two "Venoject modified" tubes. To each of one "Venoject regular" and one "Venoject modified" tubes, 4 $\mu$l of 500 IU/ml of single chain t-PA dissolved in 1.0 mol/l $KHCO_3$ is added. This results in an increase in t-PA activity by about 8.9 IU/ml in the plasma at a hematocrit of 0.45. The four tubes, "Venoject regular", "Venoject regular + t-PA", "Venoject modified" and "Venoject modified + t-PA" are incubated at room temperature (22°C) and 1 ml aliquots are drawn after 0.25, 1, 2 and 3 hours. The aliquots are centrifuged six minutes at 1500 x g and 100 $\mu$l plasma is acidified by addition of 100 $\mu$l mol/l acetate buffer pH 3.9 and analyzed according to the protocl of Wiman, *et al. Clin. Chem. Act.* 127: 279-288 (1983) using Spectrolyse/fibrin reagents from Biopool AB, Umeå. Sweden. The results of the study are shown in Table 2.

In Table 2, t-PA activity in blood plasma is measured after blood sample collection in "Venoject regular" and "Venoject modified" with and without addition of about 9 IU/ml t-PA at time zero. The blood was incubated at room temperature for 0.25, 1, 2 and 3 hours before separation of the blood cells. t-PA activity is expressed in IU/ml.

Table 2

| Time Hours | Venoject Regular | Venoject Regular and t-PA | Venoject Modified | Venoject Modified and t-PA |
|---|---|---|---|---|
| 0.25 | 0.51 | 3.8 | 1.3 | 8.4 |
| 1 | 0.14 | 1.7 | 1.6 | 9.6 |
| 2 | 0.07 | 0.6 | 1.4 | 9.6 |
| 3 | 0.04 | 0.4 | 1.4 | 9.9 |

As seen in Table 2, t-PA was stable in Venoject modified tubes while t-PA activity in unmodified tubes decreased rapidly.

**Example 3**

4.5 ml of blood is collected on 0.5 ml of (Na) citrate buffer in siliconized glass tubes from the cubital vein of sitting individuals using minimal stasis. On each of 22 different buffers (citrate buffers ranging from 0-.13 to 0.8 mol/l, pH range from approximately 3 to 5.5) blood from 5 individuals is collected. The blood is aliquoted, 3x1.5 ml, into capped polystyrene tubes, incubated at 22°C for 0.05, 1 and 4 days. Plasma is obtained by centrifugation for 10 minutes at 3000 x g, frozen and stored at - 20°C. tPA activity is determined with a fibrin stimulated chromogenic substrate assay (Biopool AB, Umeå, Sweden). The mean tPA activity half life (t1/2) is then determined. The pH is measured with a standard glass electrode. Relative degree of hemoloysis, hemolysis factor (HF), is determined as increase in absorbance at a wavelength of 540 as compared to collection on 0.13 mol/l tri-sodium citrate.

Table 3

| tPA activity m plasma (IU/ml) from blood stored at room temperature | | | | | | |
|---|---|---|---|---|---|---|
| 0.5 mol/l citrate pH 4.0 | | | 0.13 mol/. citrate | | | |
| subj. | day 0 | day 1 | day 4 | day 0 | day 1 | day 4 |
| 1 | 0.9 | 0.8 | 0.6 | 0.1 | 0 | 0 |
| 2 | 1.2 | 1.0 | 0.9 | 0.4 | 0.1 | 0 |
| 3 | 1.1 | 1.1 | 0.8 | 0.5 | 0 | 0 |
| 4. | 0.7 | 0.7 | 0.7 | 0 | 0 | 0 |
| 5 | 1.0 | 1.0 | 0.8 | 0.4 | 0 | 0 |

Table 4

| Absorbance at 540 nm in plasma from blood stored at room temperature | | | | | | |
|---|---|---|---|---|---|---|
| 0.5 mol/l citrate pH 4.0 | | | 0.13 mol/. citrate | | | |
| subj. | day 0 | day 1 | day 4 | day 0 | day 1 | day 4 |
| 1 | 1.4 | 1.7 | 2.7 | 1.1 | 1.3 | 2.1 |
| 2 | 0.2 | 0.4 | 3.2 | 0.2 | 0.7 | 0.8 |
| 3 | 0.6 | 0.7 | 3.0 | 0.8 | 0.3 | 1.3 |
| 4. | 1.9 | 1.4 | 4.9 | 1.3 | 1.1 | 2.4 |
| 5 | 0.4 | 0.3 | 3.6 | 0.8 | 1.0 | 1.1 |

The preferred buffer composition was found to be approximately 0.5 mol/l of citrate at a pH of approximately 4.0. Fig. 1 and Table 3 shows tPA activity in blood at 0.05, 1 and 4 days after collection on citrate buffer, 0.5 mol/l at pH 4.0, and conventional 0.13 mol/l tri-sodium citrate buffer. As shown in Fig. 1, the tPA in the 0.5 mol/l buffer was much more stable than tPA in the conventional buffer. The final pH of the blood sample was approximately 5.5 as compared to 8.3 with the conventional 0.13 mol/l tri-sodium citrate

EP 0 576 039 A2

buffer. The mean tPA activity half life in blood at roome temperature was 17 days when collected on the 0.5 mol/l citrate buffer at a pH of 4.0. Table 4 shows hemolysis in the two buffer systems. As shown, the hemolysis in the preferred buffer composition is acceptable.

It should be understood that the foregoing relates only to a preferred embodiment of the present invention and that numerous modifications or alterations may be made without departing from the spirit and scope of the invention as set forth in the appended claims.

**Claims**

1. A method for determining t-PA activity in biological fluids, comprising (a) mixing a biological fluid containing an unknown amount of t-PA activity with plasminogen, a plasmin substrate and a t-PA stimulator, and (b) quantitating the amount of t-PA activity in the biological fluid by measuring the cleavage of the plasmin substrate, said method being characterized by adding to the mixture antibodies which inhibit the activity of PAI 1.

2. The method of claim 1, wherein the t-PA stimulator is obtained by combining a component selected from the group consisting of poly-D-lysine, poly-L-lysine, derivatives of poly-D-lysine and derivatives of poly-L-lysine, and a component selected from the group consisting of fibrinogen, plasmin digests of fibrinogen, plasmin digests of fibrin and components of plasmin digests of fibrinogen or fibrin.

3. The method of claims 1 or 2, further comprising the addition of antibodies that inhibit the activity of alpha-2-antiplasmin.

Fig. 1